# EUROPÄISCHE PATENTSCHRIFT

(11) **EP 1 173 092 B1**
(45) Veröffentlichungstag und Bekanntmachung des Hinweises auf die Patenterteilung: **11.08.2004**
(21) Anmeldenummer: 01913840.3
(22) Anmeldetag: 26.02.2001
(51) Int. Cl.: A61B 5/024

(54) **PULSSENSOR**
PULSE MONITOR
CAPTEUR DE PULSATIONS

(30) Priorität: 24.02.2000 DE 20003390 U
(43) Veröffentlichungstag der Anmeldung: 23.01.2002
(73) Patentinhaber: Heinz Kettler GmbH & Co., 59469 Ense-Parsit (DE)
(72) Erfinder: KETTLER, Heinz, 59469 Ense-Parsit (DE)
(74) Vertreter: Weber, Joachim, Dr.
(86) Internationale Anmeldenummer: PCT/EP2001/002181
(87) Internationale Veröffentlichungsnummer: WO 2001/062146

(56) Entgegenhaltungen:
- WO-A-94/16774
- US-A- 4 319 581
- US-A- 5 544 649

## Beschreibung

Die Erfindung bezieht sich auf einen Pulssensor mit zumindest einem von Hand kontaktierbaren Kontaktbereich sowie mit elektrischen Anschlussmitteln.

Pulssensoren der beschriebenen Art werden beispielsweise bei Trainingsgeräten oder Sportgeräten verwendet. Derartige Pulssensoren unterscheiden sich von Puls-Überwachungseinrichtungen, die am Körper selbst getragen werden müssen, beispielsweise in Form eines Brustbandes. Die erfindungsgemäßen Pulssensoren sind an Griffbereichen oder Lenkern, beispielsweise von Steppern, von Fahrradtrainern oder Laufbändern montiert. Bei den bekannten Geräten werden isolierte Metallplatten an dem Träger, beispielsweise einem Rohr eines Lenkers oder eines Gestells eines Trainingsgerätes oder Ähnlichem fixiert. Die elektrischen Anschlusskabel verlaufen innerhalb dieses Rohres, sodass das Rohr sowohl mit Einlassöffnungen als auch mit Auslassöffnungen für die elektrischen Anschlussmittel oder Kabel versehen werden muss. Weiterhin ist es erforderlich, Schraublöcher oder Ähnliches zur Befestigung der Pulsabnehmer oder Pulssensoren vorzusehen. Zusätzlich ist es erforderlich, Montageöffnungen bereitzustellen, um die Verkabelung durch das Trägerrohr, das Gestell oder Ähnliches zu führen.

Insgesamt ergibt sich somit aus dem Stand der Technik, dass die Pulssensoren oder Pulsabnehmer ausgesprochen aufwendig hinsichtlich ihres Aufbaus und vor allem hinsichtlich ihrer Montage sind.

Der Erfindung liegt die Aufgabe zugrunde, einen Pulssensor der eingangs genannten Art zu schaffen, welcher bei einfachem Aufbau und einfacher, betriebssicherer Anwendbarkeit leicht zu montieren ist.

Erfindungsgemäß wird die Aufgabe durch die Merkmale des Hauptanspruchs gelöst, die Unteransprüche zeigen weitere vorteilhafte Ausgestaltungen der Erfindung.

Es ist somit vorgesehen, dass der Pulssensor ein Trägerelement umfasst, welches teilzylinderschalenartig ausgebildet ist, an seiner Oberseite den Kontaktbereich trägt und mit zumindest einer Nut zur Aufnahme der elektrischen Anschlussmittel versehen ist.

Der erfindungsgemäße Pulssensor zeichnet sich durch eine Reihe erheblicher Vorteile aus. Durch die Verwendung eines teilzylinderschalenartigen Trägerelements ist es möglich, den Pulssensor an beliebiger Stelle an einem Rohr oder Ähnlichem zu fixieren. Hierdurch vereinfacht sich zum Einen die Herstellung von Trainingsgeräten oder Ähnlichem zum Zweiten wird die Möglichkeit geschaffen, derartige Pulssensoren nachträglich anzubauen.

Durch die erfindungsgemäß vorgesehene teilzylinderschalenartige Form des Trägerelements kann dieses aufgesteckt oder aufgeclipst beziehungsweise aufgeklemmt werden. Zusätzliche Montagemaßnahmen, wie beispielsweise Löcher, Schrauben oder Ähnliches sind nicht erforderlich. Die Aufsteckung, Aufclipsung oder Aufklemmung hat weiterhin den Vorteil, dass es möglich ist, die Position des Pulssensors zu verändern. Eine Trainingsperson hat somit die Möglichkeit, die Pulssensoren an ihre gewünschte Handhaltung anzupassen, sodass die Pulssensoren in optimaler Weise gegriffen werden können.

Da die erfindungsgemäßen Pulssensoren mit zumindest einer Nut zur Aufnahme der elektrischen Anschlussmittel versehen sind, ist gewährleistet, dass das oder die Anschlusskabel in optimaler Weise geführt werden können. Hierdurch kann auch eine ausreichende Zugentlastung sichergestellt sein, sodass weitere Befestigungsmaßnahmen vollständig entfallen können.

Die Verdrahtung geschieht erfindungsgemäß bevorzugterweise durch Spiralkabel außerhalb des den Pulssensor tragenden Rohrs.

Ein weiterer, wesentlicher Vorteil des erfindungsgemäßen Pulssensors liegt darin, dass handelsübliche Kontaktbereiche, welche meist in Form von Metallplatten ausgebildet sind, verwendet werden können. Es ist somit in besonders einfacher Weise möglich, bestehende Kontaktbereiche an bestehende elektronische Schaltungen zur Weiterverarbeitung der ermittelten Signale anzuschließen. Im Bereich einer Anzeigeeinrichtung, die den jeweiligen Puls anzeigt, sind somit keine weiteren Modifikationen erforderlich.

In besonders günstiger Weiterbildung der Erfindung ist vorgesehen, dass das Trägerelement seitliche elastische Schenkel zum lösbaren Aufclipsen auf ein Rohr umfasst. Weiterhin ist es vorteilhaft, wenn an dem Trägerrohr eine unterseitige, teilzylindrische Ausnehmung vorgesehen ist, die in ihrer Größe und Dimensionierung exakt an das jeweilige Trägerrohr oder Ähnliches eines Trainingsgerätes, Fahrrades oder Ähnlichem angepasst werden kann.

Die Nut ist bevorzugterweise an der Unterseite des Trägerelements ausgebildet, sodass die Handkräfte, mittels derer der Pulssensor von einer Trainingsperson gegriffen wird, zum zusätzlichen Klemmen des Trägerelements und zum Greifen der elektrischen Kabel dienen können.

Die Nut erstreckt sich bevorzugterweise in Längsrichtung des Trägerelements, sodass dieses wahlweise beidseitig anschließbar ist. Besonders günstig ist es, wenn sich die Nut jeweils in zwei gegabelte Endnuten verzweigt. Hierdurch kann das Anschlusskabel auf besonders günstige Weise nach unten/hinten geführt werden, sodass es dem direkten Zugriff einer Trainingsperson entzogen ist und auch optisch nicht stört.

Der Kontaktbereich ist im Rahmen der Erfindung in Form eines an der Oberseite des Trägerelements angeordneten metallischen Elements ausgebildet. Es ist erfindungsgemäß auch möglich, zwei oder mehrere derartige metallische Elemente vorzusehen.

Bei einer Fertigung des Trägerelements aus einem Kunststoff wird zum Einen das formschlüssige Aufclipsen an einem Trägerrohr oder Ähnlichem erleichtert, zum Anderen isoliert das Trägerelement die Kontaktbereiche.

Im Folgenden wird die Erfindung anhand eines Ausführungsbeispiels in Verbindung mit der Zeichnung beschrieben. Dabei zeigt:
Fig. 1 eine Draufsicht auf den erfindungsgemäßen Pulssensor,
Fig. 2 eine Schnittansicht längst der Linie II-II von Fig. 1,
Fig. 3 eine Schnittansicht längs der Linie III-III von Fig. 1,
Fig. 4 eine Schnittansicht längs der Linie IV-IV von Fig. 1,
Fig. 5 eine Unteransicht des Pulssensors, und
Fig. 6 eine Schnittansicht längs der Linie VI-VI von Fig. 5.

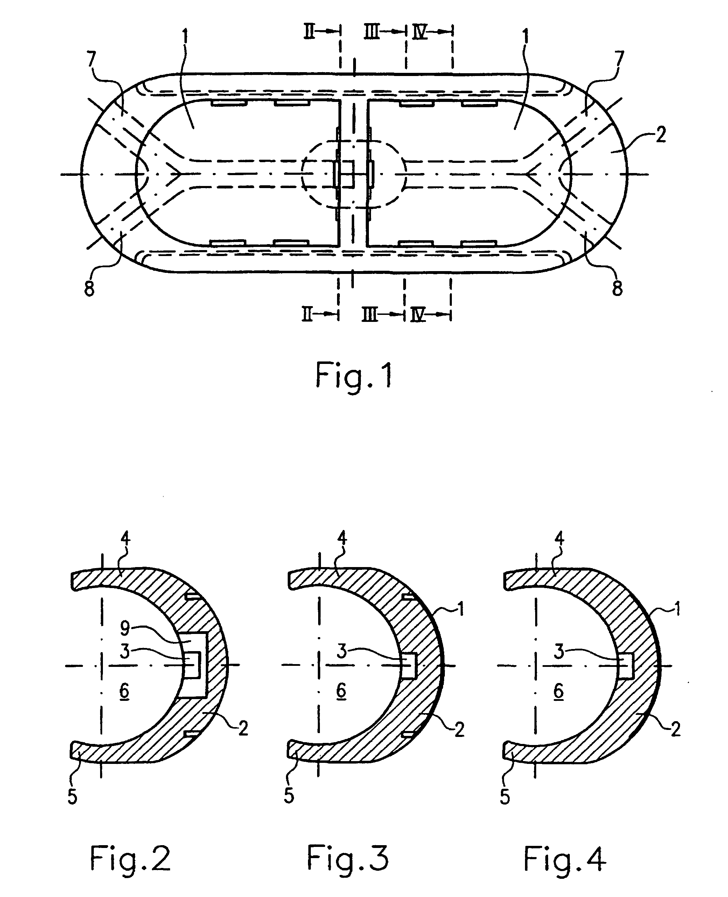

Wie sich aus den Zeichnungen ergibt, weist der erfindungsgemäße Pulssensor ein halbzylinderschalenartiges Trägerelement 2 auf, welches mit einer teilzylindrischen zentrischen Ausnehmung 6 versehen ist und zwei Schenkel 4, 5 hat. Wie sich aus den Schnittansichten der Fig. 2 bis 4 ergibt, erstrecken sich die Schenkel 4, 5 über die Mittellinie der Ausnehmung 6, sodass das Trägerelement 2, welches bevorzugterweise aus einem elastischen Kunststoff gefertigt ist, auf ein nichtdargestelltes Rohr, beispielsweise eines Fahrradtrainers, eines Laufbandes oder eines Steppers aufclipbar ist.

An seiner Außenseite weist das Trägerelement 2 einen metallischen Kontaktbereich 1 auf, welcher beispielsweise in Form einer Metallplatte ausgebildet ist. Der Kontaktbereich 1 ist fest mit dem Trägerelement 2 verbunden, beispielsweise verklebt oder eingespritzt.

An seiner Unterseite ist im zentrischen Bereich des Trägerelements 2 eine Ausnehmung 9 vorgesehen, welche zur Kontaktdurchführung und zum Anschluss der Kontaktbereiche 1 dient. Die Kontaktbelegung ist im Einzelnen nicht dargestellt, da sie dem Stand der Technik entspricht. Gleiches gilt für den Aufbau. des metallischen Kontaktbereiches.

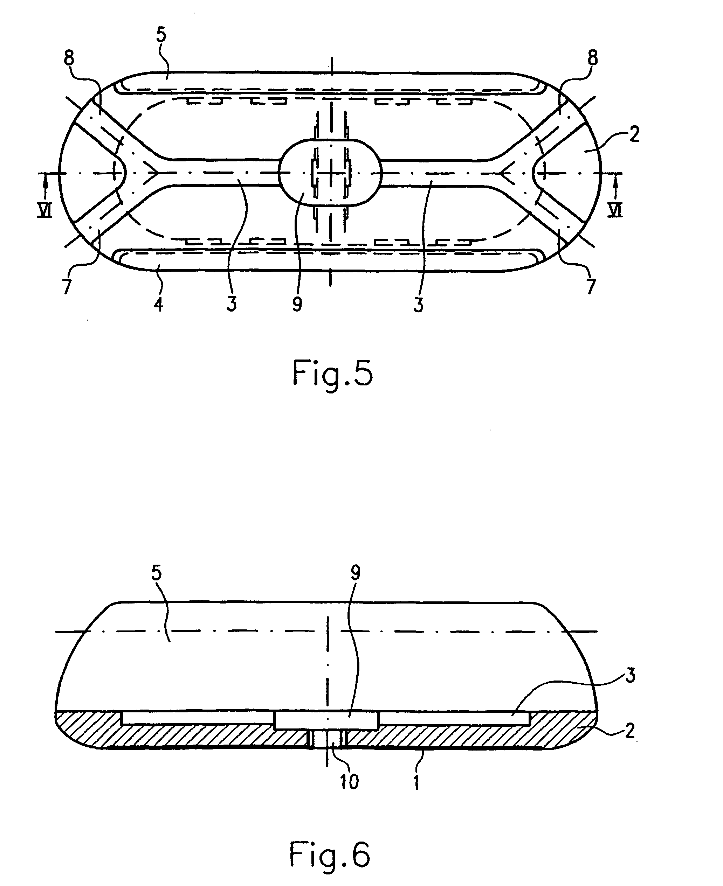

Das Trägerelement weist an seiner Unterseite eine sich in seiner Längsrichtung erstreckende Nut 3 auf, die sich an ihren Enden in jeweils zwei Endnuten 7, 8 gabelt. Durch die Nut 3 beziehungsweise die Endnuten 7, 8 kann ein Elektrokabel geführt werden, das durch die in Fig. 6 gezeigte Ausnehmung 10 an den Kontaktbereichen 1 befestigbar ist. Das Kabel kann in seinem weiteren Verlauf als Spiralkabel ausgestaltet sein. Die Form der Nuten 3, 7 und 8 kann so gewählt werden, dass das Kabel einclipsbar ist. Es ist jedoch auch verklebbar oder in anderer Weise befestigbar. Die Endnuten 7, 8, die sich seitlich gabeln, gestatten eine entsprechend ergonomische und platzsparende Abführung der Anschlusskabel.

Der erfindungsgemäße Pulssensor weist somit den Vorteil auf, dass er individuell an verschiedenen Positionen eines Trainingsgerätes angebracht werden kann. Zu seiner Montage sind keinerlei Bohrungen an dem Trägerrohr oder Trägergestell erforderlich, weiterhin ist es nicht notwendig, die Verkabelung durch das Trägerrohr oder Trägergestell zu führen.

Die Erfindung ist nicht auf das gezeigte Ausführungsbeispiel beschränkt, vielmehr ergeben sich im Rahmen der Erfindung vielfältige Abwandlungs- und Modifikationsmöglichkeiten.

## Patentansprüche

1. Pulssensor mit zumindest einem von Hand kontaktierbaren Kontaktbereich (1) sowie mit elektrischen Anschlussmitteln, wobei der Pulssensor ein Trägerelement (2) umfasst, welches an seiner Oberseite den Kontaktbereich (1) trägt und mit zumindest einer Nut (3) zur Aufnahme der elektrischen Anschlussmittel versehen ist **gekennzeichnet dadurch, dass** das Trägerelement teilzylinder schalenartig ausgebildet ist.

2. Pulssensor nach Anspruch 1, **dadurch gekennzeichnet, dass** das Trägerelement (2) seitlich elastische Schenkel (4, 5) zum lösbaren Aufclipsen auf ein Rohr umfasst.

3. Pulssensor nach Anspruch 1 oder 2, **dadurch gekennzeichnet, dass** das Trägerelement (2) eine unterseitige, teilzylindrische Ausnehmung (6) aufweist.

4. Pulssensor nach einem der Ansprüche 1 bis 3, **dadurch gekennzeichnet, dass** die Nut (3) an der Unterseite des Trägerelements (2) ausgebildet ist.

5. Pulssensor nach Anspruch 4, **dadurch gekennzeichnet, dass** sich die Nut (3) in Längsrichtung des Trägerelements (2) erstreckt.

6. Pulssensor nach Anspruch 5, **dadurch gekennzeichnet, dass** die Nut (3) jeweils in zwei gegabelte Endnuten (7, 8) übergeht.

7. Pulssensor nach einem der Ansprüche 1 bis 6, **dadurch gekennzeichnet, dass** der Kontaktbereich (1) in Form eines an der Oberseite des Trägerelements (2) angeordneten metallischen Elements ausgebildet ist.

8. Pulssensor nach Anspruch 7, **dadurch gekennzeichnet, dass** zwei metallische Elemente vorgesehen sind.

9. Pulssensor nach einem der Ansprüche 1 bis 8, **dadurch gekennzeichnet, dass** die elektrischen Anschlussmittel in Form von in der Nut (3) befestigbaren Kabeln ausgebildet sind.

10. Pulssensor nach einem der Ansprüche 1 bis 9, **dadurch gekennzeichnet, dass** das Trägerelement (2) aus Kunststoff gefertigt ist.

## Claims

1. Pulse monitor with at least one contact region (1), which may be contacted with the hand and electrical connection means, wherein the pulse monitor comprises a support element (2), on the upper side of which the contact region (1) is arranged and is provided with at least one groove (3) for accepting the electrical connection means, characterised thereby in that the support element is embodied in a part-cylinder form

2. Pulse monitor according to claim 1, **characterized in that** the support element (2) comprises lateral, flexible shanks (4, 5) for the unlockable clipping onto a tube.

3. Pulse monitor according to claims 1 or 2, **characterized in that** the support element (2) has a bottom part-cylinder recess (6).

4. Pulse monitor according to one of the claims 1 to 3, **characterized in that** the groove (3) is embodied on the bottom side of the support element (2).

5. Pulse monitor according to claim 4, **characterized in that** the groove (3) extends in the longitudinal direction of the support element (2).

6. Pulse monitor according to claim 5, **characterized in that** the groove (3) ends in two respectively forked end grooves (7, 8).

7. Pulse monitor according to one of the claims 1 to 6, **characterized in that** the contact region (1) is embodied in the form of a metallic element that is arranged on the upper side of the support element (2).

8. Pulse monitor according to claim 7, **characterized in that** two metallic elements are provided.

9. Pulse monitor according to one of the claims 1 to 8, **characterized in that** the electrical connection means are embodied in the form of cables that can be attached in the groove (3).

10. Pulse monitor according to one of the claims 1 to 9, **characterized in that** the support element (2) is manufactured of plastic material.

## Revendications

1. Capteur de pulsations avec au moins une zone de contact (1) contactable à la main ainsi qu'avec des moyens de connexion électrique, le capteur de pulsations comprenant un élément de support (2) qui porte la zone de contact (1) sur sa face supérieure et étant muni d'au moins une gorge (3) pour recevoir les moyens de connexion électrique, **caractérisé par le fait que** l'élément de support est en forme de coque partiellement cylindrique.

2. Capteur de pulsations selon la revendication 1, **caractérisé par le fait que** l'élément de support (2) comprend des branches latérales élastiques (4, 5) pour le clipsage amovible sur un tube.

3. Capteur de pulsations selon la revendication 1 ou 2, **caractérisé par le fait que** l'élément de support (2) présente un évidement partiellement cylindrique (6) sur sa face inférieure.

4. Capteur de pulsations selon l'une des revendications 1 à 3, **caractérisé par le fait que** la gorge (3) est formée sur la face inférieure de l'élément de support (2).

5. Capteur de pulsations selon la revendication 4, **caractérisé par le fait que** la gorge (3) s'étend en direction longitudinale de l'élément de support (2).

6. Capteur de pulsations selon la revendication 5, **caractérisé par le fait que** la gorge (3) se divise de chaque côté en deux gorges terminales (7, 8) en fourche.

7. Capteur de pulsations selon l'une des revendications 1 à 6, **caractérisé par le fait que** la zone de contact (1) est réalisée sous la forme d'un élément métallique situé sur la face supérieure de l'élément de support (2).

8. Capteur de pulsations selon la revendication 7, **caractérisé par le fait qu'**il est prévu deux éléments métalliques.

9. Capteur de pulsations selon l'une des revendications 1 à 8, **caractérisé par le fait que** les moyens de connexion électrique sont réalisés sous la forme de câbles pouvant être fixés dans la gorge (3).

10. Capteur de pulsations selon l'une des revendications 1 à 9, **caractérisé par le fait que** l'élément de support (2) est réalisé en matière synthétique.
